# EUROPÄISCHE PATENTSCHRIFT

(11) **EP 2 952 502 B1**
(45) Veröffentlichungstag und Bekanntmachung des Hinweises auf die Patenterteilung: **04.01.2017**
(21) Anmeldenummer: 14171202.6
(22) Anmeldetag: 04.06.2014
(51) Int. Cl.: C07C 267/00, C08K 5/29, C08L 75/06, C08G 18/02, C08G 18/81

(54) **BIS[3 ISOPROPENYL-ALPHA,ALPHA-DIMETHYLBENZYL]CARBODIIMID, VERFAHREN ZUR HERSTELLUNG UND DESSEN VERWENDUNG**
BIS[3 ISOPROPENYL-ALPHA, ALPHA-DIMETHYLBENZYL] CARBODIIMIDE, METHOD FOR ITS PRODUCTION AND ITS USE
CYANAMIDE BIS[3 ISOPROPENYL-ALPHA,ALPHA-DIMÉTHYL BENZYLE], PROCÉDÉ DE FABRICATION ET SON UTILISATION

(43) Veröffentlichungstag der Anmeldung: 09.12.2015
(73) Patentinhaber: Rhein Chemie Rheinau GmbH, 68219 Mannheim (DE)
(72) Erfinder: Laufer, Wilhelm, 67158 Ellerstadt (DE); Eckert, Armin, 68794 Oberhausen-Rheinhausen (DE); Schönhaber, Martina, 64287 Darmstadt (DE); Herd, Oliver, 42281 Wuppertal (DE); Sperber, Rolf, 42329 Wuppertal (DE)
(74) Vertreter: Siegers, Britta

(56) Entgegenhaltungen:
- WO-A1-2005/111136
- WO-A2-93/22282

## Beschreibung

Die Erfindung betrifft neue Verfahren zur Herstellung von Bis[3-Isopropenyl-α,α-dimethylbenzyl]carbodiimid, das darüber hergestellte Bis[3-Isopropenyl-α,α-dimethylbenzyl]-carbodiimid und dessen Verwendung als Hydrolyseschutzmittel in Polyurethan(PU)-basierten Systemen, vorzugsweise thermoplastisches TPU, PU-Klebstoffen, PU-Gießharzen, PU-Elastomeren oder PU-Schäumen.

Carbodiimide haben sich in vielen Anwendungen bewährt, z.B. als Hydrolyseschutzmittel für thermoplastische Kunststoffe, Polyole, Polyurethane, Triglyceride und Schmierstofföle etc.

Die Synthese der Carbodiimide erfolgt nach dem Stand der Technik ausgehend von Isocyanaten die durch basische oder heterocyclische Katalyse unter CO₂-Abspaltung carbodiimidisiert werden. Dabei können mono- oder polyfunktionelle Isocyanate zu monomeren oder polymeren Carbodiimiden umgesetzt werden.

Die üblicherweise verwendeten Katalysatoren sind Alkali- oder Erdalkaliverbindungen, sowie heterocyclische Verbindungen, die Phosphor enthalten, siehe Angew. Chem. 1962, 74, 801-806 und Angew. Chem. 1981, 93, 855-866.

Die Herstellung von Bis[3-Isopropenyl-α,α-dimethylbenzyl]carbodiimid ist schwierig, da das Endprodukt oft nur in geringer Ausbeute erzielt werden kann, siehe WO-A 2005/111136, in der die Synthese des Bis[3-Isopropenyl-α,α-dimethylbenzyl]carbodiimids, ausgehend von 3-Isopropenyl-α,α-dimethylbenzylisocyanat mit der heterocyclischen Phosphorverbindung 1-Methyl-2-phospholen-1-oxid (MPO) als Katalysator beschrieben wird, und lediglich eine Ausbeute von 37 % erzielt wird.

Zudem schwierig ist die vollständige Entfernung des in der Regel verwendeten Phosphor-haltigen Katalysators. Da Carbodiimide vorzugsweise bei der Herstellung von Polyurethanen eingesetzt werden, ist die Anwesenheit von Phosphor, selbst im Spurenbereich, extrem störend und ist daher zu vermeiden.

Die bisher bekannten Synthesen sind somit nicht wirtschaftlich, so dass Bis[3-Isopropenyl-α,α-dimethylbenzyl]carbodiimid nicht kommerziell für den Hydrolyseschutz von Polyestern und esterbasierten PU-Systemen zur Verfügung gestellt und eingesetzt werden kann.

Aufgabe der vorliegenden Erfindung war es daher, ein verbessertes Verfahren bereitzustellen, welches die Herstellung von Bis[3-Isopropenyl-α,α-dimethylbenzyl]carbodiimid in hohen Ausbeuten ermöglicht und zudem zu einem Bis[3-Isopropenyl-α,α-dimethylbenzyl]carbodiimid führt, das frei von organischen Phosphorverbindungen ist, so dass dies bei der Herstellung und/oder Stabilisierung von PU-Systemen eingesetzt werden kann.

Überraschenderweise wurde nun gefunden, dass diese vorgenannten Aufgaben erfüllt sind, wenn Bis[3-Isopropenyl-α,α-dimethylbenzyl]carbodiimid durch die Umsetzung von 3-Isopropenyl-α,α-dimethylbenzylisocyanat in Gegenwart von 0,1 - 20 Gew.% basischer Cäsiumsalze als Katalysator bei Temperaturen zwischen 160 bis 220° C unter Kohlendioxidabspaltung umgesetzt (carbodiimidisiert) wird.

Gegenstand der vorliegenden Erfindung ist daher ein Verfahren zur Herstellung von Bis[3-Isopropenyl-α,α-dimethylbenzyl]carbodiimid, wonach 3-Isopropenyl-α,α-dimethyl-benzylisocyanat in Gegenwart von 0,1 - 20 Gew.%, vorzugsweise 0,5 - 10 Gew.%, besonders bevorzugt 1 - 5 Gew.% an basischen Cäsiumssalzen bei Temperaturen zwischen 160 bis 220° C, bevorzugt 180 bis 210°C, besonders bevorzugt 190 bis 200°C, carbodiimidisiert wird.

Als basische Cäsiumssalze im Sinne der Erfindung werden vorzugsweise Cäsiumcarbonat und/oder Cäsiumalkoholat, vorzugsweise Cäsiummethylat und/oder Cäsiumethylat eingesetzt.

In einer bevorzugten Ausführungsform des erfindungsgemäßen Verfahrens werden die basischen Cäsiumssalze im Anschluss an die Carbodiimidisierung abfiltriert.

In einer weiteren bevorzugten Ausführungsform des erfindungsgemäßen Verfahrens findet die Carbodiimisierung in einem Lösemittel statt.

Als Lösemittel werden bevorzugt ein-, zwei-, drei- oder mehrfach, vorzugsweise zweifach Alkyl-substituierte Benzole und/oder Dibenzole eingesetzt, mit Alkyl = C₁ - C₃. Als Alkylbenzole sind Xylole besonders bevorzugt.

Ein weiterer Gegenstand der vorliegenden Erfindung ist das Bis[3-Isopropenyl-α,α-dimethylbenzyl]carbodiimid erhältlich nach dem erfindungsgemäßen Verfahren, welches vorzugsweise einen Anteil an heterocyclischen Phosphorverbindungen, insbesondere 1-Methyl-2-phospholen-1-oxid (MPO), von weniger als 1 ppm aufweist und welches besonders bevorzugt frei von heterocyclischen Phosphorverbindungen ist.

Gegenstand der vorliegenden Erfindung ist somit auch ein Stabilisator, enthaltend mindestens 90 Gew.% Bis[3-Isopropenyl-α,α-dimethylbenzyl]carbodiimid, der nach dem erfindungsgemäßen Verfahren hergestellt wurde und maximal 1 ppm an heterocyclischen Phosphorverbindungen enthält.

### Bis[3-Isopropenyl-α,α-dimethylbenzyl]carbodiimid entspricht dabei der nachstehend genannten Formel:

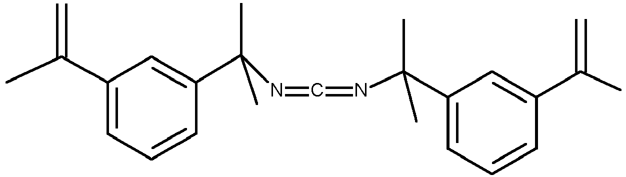

Ein weiterer Gegenstand der vorliegenden Erfindung ist zudem ein Verfahren zur Herstellung von Polyurethanen (PU), bevorzugt thermoplastischen Polyurethanen, wonach die Umsetzung der Polyole, vorzugsweise der Polyesterpolyole, mit den Diisocyanaten in Gegenwart des erfindungsgemäßen Bis[3-Isopropenyl-α,α-dimethylbenzyl]carbodiimids gegebenenfalls in Anwesenheit von Katalysatoren und gegebenenfalls weiterer Hilfs- und Zusatzstoffe durchgeführt wird.

Die Herstellung der Polyurethane erfolgt dabei vorzugsweise, wie in WO 2005/111136 A1 beschrieben, unter Verwendung des erfindungsgemäßen Bis[3-Isopropenyl-α,α-dimethylbenzyl]-carbodiimids als Stabilisator.Polyurethane entstehen durch Polyadditionsreaktion von Polyisocyanaten mit mehrwertigen Alkoholen, den Polyolen, nahezu quantitativ. Die Verknüpfung erfolgt durch die Reaktion einer Isocyanatgruppe (-N=C=O) eines Moleküls mit einer Hydroxygruppe (-OH) eines anderen Moleküls unter Bildung einer Urethangruppe (-NH-CO-O-).

Thermoplastische Polyurethane sind dabei unter Temperatureinwirkung verformbare Polyurethane.

Der Reaktionsverlauf zwischen Diisocyanat und Polyol ist abhängig von dem Molverhältnis der Komponenten. Zwischenstufen mit gewünschtem Durchschnittsmolekulargewicht und gewünschten Endgruppen können durchaus erhalten werden. Diese Zwischenstufen können dann zu einem späteren Zeitpunkt mit einem Diol oder Diamin umgesetzt (kettenverlängert) werden, wobei dann das gewünschte Polyurethan bzw. Polyurethan-Polyharnstoff-Hybrid gebildet wird. Die Zwischenstufen werden im Allgemeinen als Prepolymer bezeichnet.

Das Molverhältnis zwischen Diisocyanat und Polyol liegt vorzugsweise zwischen 1 : 2 bis 10 : 1. Geeignete Polyole für die Herstellung von Prepolymeren sind Polyalkylenglykolether, Polyetherester oder Polyester mit endständigen Hydroxylgruppen (Polyesterpolyole).

Bei den Polyolen im Sinne der Erfindung handelt es sich um Verbindungen, die vorzugsweise ein Molekulargewicht in (g/mol) von bis zu 2000, bevorzugt im Bereich von 500 bis 2000 und besonders bevorzugt im Bereich von 500 bis 1000 aufweisen.

Der Begriff Polyol im Sinne der Erfindung umfasst dabei sowohl Diole als auch Triole, wie auch Verbindungen mit mehr als drei Hydroxylgruppen je Molekül. Die Verwendung von Triolen ist besonders bevorzugt.

Bevorzugte Polyole sind Polyesterpolyole und/oder Polyetheresterpolyole.

Vorteilhaft ist es, wenn das Polyol eine OH-Zahl von bis zu 200, vorzugsweise zwischen 20 und 150 und besonders bevorzugt zwischen 50 und 115, aufweist.

Insbesondere eignen sich Polyesterpolyole, die Reaktionsprodukte von verschiedenen Polyolen mit aromatischen oder aliphatischen Dicarbonsäuren und/oder Polymeren von Lactonen sind.

Bevorzugt hierbei sind aromatische Dicarbonsäuren, welche zur Bildung geeigneter Polyesterpolyole verwendet werden können. Besonders bevorzugt sind hier Terephthalsäure, Isophthalsäure, Phthalsäure, Phtalsäureanhydrid sowie substituierte Dicarbonsäureverbindungen mit Benzolkern.

Als aliphatische Dicarbonsäuren sind solche bevorzugt, welche zur Bildung geeigneter Polyesterpolyole verwendet werden können, besonders bevorzugt Sebacinsäure, Adipinsäure und Glutarsäure.

Als Polymere von Lactonen sind solche bevorzugt, welche zur Bildung geeigneter Polyesterpolyole verwendet werden können, besonders bevorzugt Polycaprolacton.

Sowohl bei den Dicarbonsäuren als auch bei den Polymeren von Lactonen handelt es sich um handelsübliche Substanzen.

Besonders bevorzugt sind auch solche Polyole, welche zur Bildung geeigneter Polyesterpolyole verwendet werden können, ganz besonders bevorzugt Ethylenglykol, Butandiol, Neopentylglykol, Hexandiol, Propylenglykol, Dipropylenglykol, Diethylenglykol und Cyclohexandimethanol.

In einer weiteren bevorzugten Ausführungsform der Erfindung handelt es sich bei den Polyolen um Polyetheresterpolyole.

Hierfür sind die Reaktionsprodukte von verschiedenen vorhergehend genannten Polyolen mit aromatischen oder aliphatischen Dicarbonsäuren und/oder Polymeren von Lactonen, vorzugsweise Polycaprolacton, bevorzugt.

Bei den im Sinne der Erfindungen eingesetzten Polyolen handelt es sich um handelsübliche Verbindungen, die bei der Firma Bayer MaterialScience AG unter dem Handelsnamen Baycoll^{®} oder Desmophen^{®} erhältlich sind.

Als Diisocyanate sind aromatische und aliphatische Diisocyanate bevorzugt. Besonders bevorzugt sind Toluol-2,4-diisocyanat, Toluol-2,6-diisocyanat, Phenylendiisocyanat, 4,4-Diphenylmethandiisocyanat, Methylen-bis(4-phenylisocynat), Naphtalen-1,5-diisocyanat, Tetramethylen-1,4-diisocyanat und/oder Hexamethylen-1,6-diisocyanat, ganz besonders bevorzugt Toluol-2,4-diisocyanat und Toluol-2,6-diisocyanat.

Bei den im Sinne der Erfindungen eingesetzten Diisocyanaten handelt es sich um handelsübliche Verbindungen, die bei der Firma Bayer MaterialScience AG unter dem Handelsnamen Desmodur^{®} erhältlich sind.

In einer weiteren Ausführungsform der Erfindung enthält die Zusammensetzung zusätzlich mindestens ein Diamin und/oder Diol.

Als Diamine, welche für die Kettenverlängerung eingesetzt werden, sind 2-Methylpropyl-3,5-diamino-4-chlorobenzoat, Bis-(4,4'-amino-3-chlorophenyl)-methan, 3,5-Dimethylthio-2,4-toluylendiamin, 3,5-Dimethylthio-2,4-toluylendiamin, 3,5-Diethyl-2,4-toluylendiamin, 3,5-Diethyl-2,6-toluylendiamin, 4,4'-Methylen-bis-(3-chloro-2,6-diethylanilin) und 1,3-Propandiol-bis(4-aminobenzoat) bevorzugt.

Als Diole sind Butandiol, Neopentylglykol, Hexandiol, Propylenglykol, Dipropylenglykol, Diethylenglykol und/oder Cyclohexandimethanol bevorzugt.

Bei den im Sinne der Erfindung zur Kettenverlängerung eingesetzten Diaminen oder Diolen handelt es sich um handelsübliche Verbindungen, die bei der Firma Rheinchemie Rheinau GmbH unter dem Handelsnamen Addolink^{®} erhältlich sind.

Der Anteil an Diamin und/oder Diol beträgt vorzugsweise 1 bis 20 Gew.%, bezogen auf die Gesamtmischung.

Der Begriff Gesamtmischung steht hierbei für die Summe der Bestandteile der Mischung zur Herstellung der Polyurethane.

Als Katalysatoren werden vorzugsweise Dibutylzinndilaurate oder Triethylendiamin in Dipropylenglycol eingesetzt.

Bei den im Sinne der Erfindungen eingesetzten Katalysatoren handelt es sich um handelsübliche Verbindungen, die bei der Fa. Rhein Chemie Rheinau GmbH unter dem Handelsnamen Addocat^{®} erhältlich sind.

Der Anteil an Katalysator beträgt vorzugsweise 0,1 bis 5 Gew.%, bezogen auf die Gesamtmischung.

In einer weiteren bevorzugten Ausführungsform der Erfindung werden weitere Hilfs- und Zusatzstoffe, wie vorzugsweise Entformungshilfsmittel, Flammschutzmittel, UV-Stabilisatoren und Weichmacher zugegeben.

In einer besonders bevorzugten Ausführungsform der vorliegenden Erfindung wird das Bis[3-Isopropenyl-α,α-dimethylbenzyl]carbodiimid in einer Menge von 0,1 bis 2 Gew.%, bevorzugt 0,5 bis 1 Gew.%, bezogen auf die Gesamtmischung, eingesetzt.

In einer weiteren bevorzugten Ausführungsform der vorliegenden Erfindung wird das Bis[3-Isopropenyl-α,α-dimethylbenzyl]carbodiimid in flüssiger Form (Aggregatzustand flüssig) bevorzugt bei Temperaturen von 20 - 50 °C, besonders bevorzugt 25 - 35 °C eingesetzt.

Die Zudosierung des erfindungsgemäßen Bis[3-Isopropenyl-α,α-dimethylbenzyl]carbodiimids kann sowohl bei der Herstellung des Polyurethans erfolgen als auch nachträglich dem Polyurethan über Mischaggregate zudosiert werden.

Die Flüssigdosierung im erfindungsgemäßen Verfahren erfolgt auf diskontinuierlich oder kontinuierlich, vorzugsweise auf kontinuierlich arbeitenden Verarbeitungsmaschinen, wie z.B. Ein-, Zwei- und Mehrwellenextrudern, kontinuierlich arbeitenden Co-Knetern (Typ Buss) und/ oder diskontinuierlich arbeitenden Knetern, z.B. vom Typ Banbury und anderen in der Polymerindustrie üblichen Aggregaten. Diese kann gleich zu Beginn oder im Verlauf der Herstellung des estergruppen-haltigen Polymeren oder gleich zu Beginn oder im Verlauf der Verarbeitung, z.B. zu Monofilamenten oder zu Polymergranulat, erfolgen.

Unter flüssigdosiert im Sinne der Erfindung wird verstanden, dass die erfindungsgemäßen Carbodiimide in flüssiger Form (in flüssigem Aggregatzustand) gravimetrisch oder volumetrisch den kontinuierlich oder diskontinuierlich arbeitenden Verarbeitungsmaschinen zudosiert werden. Um dies zu ermöglichen, müssen die erfindungsgemäßen Carbodiimide bei der Dosierung flüssig und niederviskos sein, insbesondere bei Umgebungstemperatur, wie sie bei der Polymerverarbeitung üblich ist. Zur Flüssigdosierung in Verarbeitungsprozessen werden die in der Thermoplast-Compoundiertechnologie üblichen, kontinuierlich arbeitenden Dosieraggregate verwendet. Diese können beheizbar sein. Bevorzugt sind diese nicht beheizbar.

Ein weiterer Gegenstand der vorliegenden Erfindung ist zudem die Verwendung des erfindungsgemäßen Bis[3-Isopropenyl-α,α-dimethylbenzyl]carbodiimids zur Stabilisierung von Polyurethanen.

Ein weiterer Gegenstand der vorliegenden Erfindung ist die Verwendung des erfindungsgemäßen Bis[3-Isopropenyl-α,α-dimethylbenzyl]carbodiimids zur Stabilisierung von Polyurethanen durch Einarbeiten in das bereits hergestellte Polyurethan mittels Flüssigdosierung bevorzugt bei Temperaturen von 20 - 50 °C, besonders bevorzugt 25 - 35 °C.

Die nach diesem Verfahren hergestellten Polyurethan(PU)-basierten Systeme zeichnen sich durch eine erhöhte Hydrolysestabilität aus.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die nachfolgenden Beispiele dienen der Erläuterung der Erfindung, ohne dabei limitierend zu wirken.

### Ausführungsbeispiele:

Beispiel 1: Herstellung von Bis[3-Isopropenyl-α,α-dimethylbenzyl]carbodiimid auf Basis des Isocyanats 3-Isopropenyl-α,α-dimethylbenzylisocyanat erfindungsgemäß mit Cäsiumcarbonat.

Beispiele 2 - 6: Herstellung von Bis[3-Isopropenyl-α,α-dimethylbenzyl]carbodiimid auf Basis des Isocyanats 3-Isopropenyl-α,α-dimethylbenzylisocyanat mit anderen Katalysatoren als Vergleichsbeispiele.

### Allgemeine Herstellvorschrift für die Beispiele 1 - 5:

Es wurden 30 g 3-Isopropenyl-α,α-dimethylbenzylisocyanat in einen 100 ml-Dreihalskolben eingewogen, der mit Innenthermometer, Rückflusskühler und Schutzgaseinlass ausgestattet war, und daraufhin wurden 0.6 g (2 Gew.%) des jeweiligen Katalysators gemäß Tabelle 1 zugefügt. In der Aufheizphase wurde ein Argonstrom als Schutzgas über die Dampfphase geleitet. Bei beginnender CO₂-Entwicklung wurde das Schutzgas abgestellt. Man ließ 3.5 h bei 195 °C (Beispiel 1-5) kräftig rühren und filtrierte anschließend das auf ca. 100°C abgekühlte Reaktionsgemisch. Die Ausbeute wurde mittels ¹H-NMR-Spektroskopie bestimmt (400 MHz, CDCl₃)

Beispiel 6 entspricht dem Beispiel 1 aus WO-A 2005/111136.

**Tabelle 1: Ausbeuten der Synthese des Carbodiimids auf Basis des Isocyanats 3-Isopropenyl-α,α-dimethylbenzylisocyanat (aus NMR).**

| Beispiel | Katalysator | T [°C] | Carbodiimid [%] | Isocyanat [%] | Nebenprodukt [%] |
|---|---|---|---|---|---|
| 1 (erf.) | Cäsiumcarbonat | 195 | 93 | 0 | 7 |
| 2 (V) | Strontiumcarbonat | 195 | 0 | 100 | 0 |
| 3 (V) | Kaliumcarbonat | 195 | 0 | 100 | 0 |
| 4 (V) | Lithiumcarbonat | 195 | 0 | 100 | 0 |
| 5 (V) | Calciumcarbonat | 195 | 0 | 100 | 0 |
| 6 (V) | Phospholenoxid | 180 | 37 | Nicht bestimmt | Nicht bestimmt |

| | | | | | |
|---|---|---|---|---|---|
| V = Vergleichsbeispiel; erf. = erfindungsgemäß | | | | | |

Die Alkalimetalle des Lithiums und des Kaliums sowie die Erdalkalicarbonate des Calciums und Strontiums haben sich, wie in Tabelle 1 ersichtlich, als völlig unbrauchbar in Bezug auf den Einsatz als Katalysator für die Carbodiimidisierung von 3-Isopropenyl-α,α-dimethylbenzylisocyanat in Bis[3-Isopropenyl-α,α-dimethylbenzyl]carbodiimid erwiesen.

Überraschenderweise zeigt das Cäsiumcarbonat hingegen für die Carbodiimidisierung eine hohe Katalysatoraktivität und führt zu Ausbeuten von über 90% und ist damit deutlich besser als die Synthese über Phospholenoxid.

Zudem kann der erfindungsgemäße Katalysator einfach durch Filtration abgetrennt werden, während im Fall des Phospholenoxides als Katalysator eine aufwändige Destillation im Vakuum zur Abtrennung erfolgen muss, so dass die Ausbeute durch diese Aufarbeitung weiter verringert wird.

### Hydrolyseschutz in TPU

Dazu wurden 1.0 Gew.% bzw. 1.5 Gew.% des erfindungsgemäßen Carbodiimids (CDI erf.) sowie der kommerziell erhältlichen Carbodiimide:
CDI 1= ein monomeres aromatisches Carbodiimid, subsituiert mit Alkylgruppen,
CDI 2 = ein polymeres aromatisches Carbodiimid,
in das kommerziell erhältliche Polyester-basierte thermoplastisches Polyurethan-Elastomer (TPU/Desmopan® 2587A) durch Extrusion mittels eines Zweischneckenextruders eingearbeitet.

Mittels Spritzgussverfahren wurden daraus Prüfkörper hergestellt, die 16 h bei 80°C nachgetempert wurden. Danach wurden sie bei 80°C in Wasser gelagert und in regelmäßigen Abständen die Zugfestigkeit geprüft.

Tabelle 2 zeigt die prozentuale relative Zugfestigkeit beginnend bei Tag 0 mit 100 %.

**Tabelle 2:**

| | Referenzmaterial | 1x extrudiert | CDI 1 1.5 % | CDI 2 1.5% | CDI erf. 1.5% | CDI erf. 1.0 % |
|---|---|---|---|---|---|---|
| Tag 0 | 100 | 100 | 100 | 100 | 100 | 100 |
| Tag 5 | 82 | 85 | 83 | 94 | 100 | n.b. |
| Tag 8 | 63 | 62 | n.b. | n.b. | 91 | n.b. |
| Tag 14 | 18 | 18 | n.b. | n.b. | 86 | n.b. |
| Tag 19 | 7 | 7 | 79 | 90 | 83 | n.b. |
| Tag 26 | 0 | 0 | 76 | 88 | 82 | n.b. |
| Tag 39 | | | 72 | 88 | 82 | n.b. |
| Tag 45 | | | 63 | 82 | 76 | 91 |
| Tag 53 | | | 57 | 88 | 73 | n.b. |
| Tag 57 | | | 42 | 86 | 76 | n.b. |
| Tag 63 | | | 21 | 64 | 76 | n.b. |
| Tag 67 | | | 0 | 13 | 73 | n.b. |
| Tag 80 | | | | | n.b. | 84 |
| Tag 94 | | | | | n.b. | 80 |
| Tag 98 | | | | | 68 | n.b. |
| Tag 105 | | | | | 71 | n.b. |
| Tag 112 | | | | | 66 | n.b. |
| Tag 118 | | | | | n.b. | 81 |
| Tag 140 | | | | | 61 | n.b. |
| Tag 143 | | | | | n.b. | 73 |
| Tag 171 | | | | | n.b. | 76 |
| Tag 192 | | | | | n.b. | 62 |
| Tag 196 | | | | | 55, *) | n.b. |
| Tag 213 | | | | | n.b. | 5 |

| | | | | | | |
|---|---|---|---|---|---|---|
| V = Vergleichsbeispiel; erf. = erfindungsgemäß, n.b. = nicht bestimmt, *) danach waren keine Prüfkörper mehr vorhanden | | | | | | |

Die Ergebnisse in der Tabelle 2 zeigen, dass das nach dem erfindungsgemäßen Verfahren hergestellte Bis[3-Isopropenyl-α,α-dimethylbenzyl]carbodiimid eine herausragende Hydrolyseschutzwirkung in thermoplastischem PU (TPU) aufweist und den Carbodiimiden des Standes der Technik überlegen ist.

### Herstellung und Farbstabilität von esterbasierten PU-Schmelzklebstoffen (Hotmelts)

### Beispiel 7:

Es wurde ein Schmelzklebstoff auf Basis von Dynacoll® 7360, ein linearer Copolyester mit primären Hydroxylfunktionen und einem mittleren molekularen Gewicht von 3500 g/mol, erhältlich von der Firma Evonik AG hergestellt und dieser wurde wie folgt additiviert:
(A) 2 Gew. % Bis[3-Isopropenyl-α,α-dimethylbenzyl]carbodiimid, hergestellt nach dem erfindungsgemäßen Verfahren,
(B) 2 Gew.% Bis[3-Isopropenyl-α,α-dimethylbenzyl]carbodiimid, hergestellt nach dem Verfahren aus WO-A 2005/111136.

Alle Mengenangaben sind in Gew.-%.

Der Schmelzklebstoff wurde wie folgt hergestellt:
Zunächst wird der Copolyester Dynacoll®7360 für 30 Minuten und bei 120 °C evakuiert. Anschließend erfolgt die Zugabe von 11,67 Gew.% Diphenylmethandiisocyanat (MDI), bezogen auf die Gesamtformulierung, und es wird für 60 Minuten bei 120 °C umgesetzt.

Anschließend wurden die Additive in den Schmelzklebstoff eingearbeitet und eine Einwirkzeit der Additive von 1 Stunde sichergestellt.

Die so hergestellten und additivierten Schmelzklebstoffe (Hot-Melt) wurden in einer Kartusche einer Temperaturalterung bei 130 °C für 48 Stunden unterworfen. Dazu wurde sie in eine Alukartusche (Licht- und Feuchtigkeitsdicht) abgefüllt und im Umluftofen für 48 Stunden bei 130 °C gealtert.

Nach der Alterung wurden die Farbe und das Schäumverhalten der Proben visuell beurteilt.

Die Ergebnisse der Messungen sind in der Tabelle 3 zusammengestellt:

**Tabelle 3:**

| **Carbodiimid** | **Farbe** | **Schäumverhalten** |
|---|---|---|
| Beispiel 7A (erf.) | Farblos bis sehr leicht gelb | Kein Schaum, keine oder sehr geringe Blasenbildung |
| Beispiel 7B (V) | Gelb-rötlich bis rot- braun | Schaum- bzw. starke Blasenbildung |

| | | |
|---|---|---|
| V = Vergleichsbeispiel; erf. = erfindungsgemäß | | |

### Resümee:

Diese Versuche zeigen, dass durch die Verwendung des erfindungsgemäßen Carbodiimides keine nennenswerten störenden Nebeneffekte bezüglich Verfärbungen und Schaumbildung auftreten. Im Vergleich dazu zeigen Carbodiimide, deren Synthese mit Phospholenoxid katalysiert wurde und die noch Spuren von Phosphor-organischen Verbindungen beinhalten, die entsprechend angegebenen Nachteile von Verfärbung und Schaumbildung.

## Patentansprüche

1. Verfahren zur Herstellung von Bis[3-Isopropenyl-α,α-dimethylbenzyl]carbodiimid, **dadurch gekennzeichnet, dass** 3-Isopropenyl-α,α-dimethylbenzylisocyanat in Gegenwart von 0,1 - 20 Gew.% basischer Cäsiumssalze bei Temperaturen zwischen 160 bis 220° C carbodiimidisiert wird.

2. Verfahren nach Anspruch 1, **dadurch gekennzeichnet, dass** als basische Cäsiumssalze Cäsiumcarbonat und/oder Cäsiumalkoholat, vorzugsweise Cäsiummethylat und/oder Cäsiumethylat eingesetzt wird.

3. Verfahren nach Anspruch 1 oder 2, **dadurch gekennzeichnet, dass** die basischen Cäsiumssalze im Anschluss an die Carbodiimidisierung abfiltiriert werden.

4. Verfahren nach einem der Ansprüche 1 bis 3, **dadurch gekennzeichnet, dass** die Carbodiimisierung in einem Lösemittel stattfindet.

5. Verfahren nach Anspruch 4, **dadurch gekennzeichnet, dass** als Lösemittel ein-, zwei-, drei- oder mehrfach Alkyl-substituierte Benzole und/oder Dibenzole mit Alkyl = C₁ - C₃ eingesetzt werden.

6. Verfahren nach Anspruch 5, **dadurch gekennzeichnet, dass** als Alkylbenzole Xylole eingesetzt werden.

7. Bis[3-Isopropenyl-α,α-dimethylbenzyl]carbodiimid erhältlich nach einem Verfahren nach einem der Ansprüche 1 bis 6.

8. Stabilisator enthaltend mindestens 90 Gew.% Bis[3-Isopropenyl-α,α-dimethylbenzyl]carbodiimid nach Anspruch 7 und maximal 1 ppm an heterocyclischen Phosphorverbindungen.

9. Verfahren zur Herstellung von Polyurethanen, bevorzugt thermoplastischen Polyurethanen **dadurch gekennzeichnet, dass** die Umsetzung der Polyole mit den Diisocyanaten in Gegenwart von Bis[3-Isopropenyl-α,α-dimethylbenzyl]carbodiimid nach Anspruch 7 durchgeführt wird.

10. Verfahren nach Anspruch 9, **dadurch gekennzeichnet, dass** das Bis[3-Isopropenyl-α,α-dimethylbenzyl]carbodiimid in einer Menge von 0,1 bis 2 Gew.%, bevorzugt 0,5 bis 1 Gew.%, bezogen auf die Gesamtmischung, eingesetzt wird.

11. Verfahren nach Anspruch 9 und 10, **dadurch gekennzeichnet, dass** das Bis[3-Isopropenyl-α,α-dimethylbenzyl]carbodiimid bevorzugt in flüssiger Form kontinuierlich oder diskontinuierlich bei Temperaturen 20 - 50 °C besonders bevorzugt 25 - 35 °C zudosiert wird.

12. Verwendung von Bis[3-Isopropenyl-α,α-dimethylbenzyl]carbodiimid nach Anspruch 7 zum Hydrolyseschutz.

## Claims

1. Process for producing bis[3-isopropenyl-α,α-dimethylbenzyl]carbodiimide, **characterized in that** 3-isopropenyl-α,α-dimethylbenzylisocyanate is carbodiimidized in the presence of 0.1 - 20 wt% of basic cesium salts at temperatures between 160°C to 220°C.

2. Process according to Claim 1, **characterized in that** the basic cesium salts employed are cesium carbonate and/or cesium alkoxide, preferably cesium methoxide and/or cesium ethoxide.

3. Process according to Claim 1 or 2, **characterized in that** the basic cesium salts are filtered off following the carbodiimidization.

4. Process according to any of Claims 1 to 3, **characterized in that** the carbodiimidization takes place in a solvent.

5. Process according to Claim 4, **characterized in that** mono-, di-, tri- or polyalkyl-substituted benzenes and/or dibenzenes where alkyl - C₁ - C₃ are employed as solvent.

6. Process according to Claim 5, **characterized in that** the alkylbenzenes employed are xylenes.

7. Bis[3-isopropenyl-α,α-dimethylbenzyl]carbodiimide obtainable by a process according to any of Claims 1 to 6.

8. Stabilizer comprising at least 90 wt% of bis[3-isopropenyl-α,α-dimethylbenzyl]carbodiimide according to Claim 7 and not more than 1 ppm of heterocyclic phosphorus compounds.

9. Process for producing polyurethanes, preferably thermoplastic polyurethanes, **characterized in that** the reaction of the polyols with the diisocyanates is performed in the presence of bis[3-isopropenyl-α,α-dimethylbenzyl]carbodiimide according to Claim 7.

10. Process according to Claim 9, **characterized in that** the bis[3-isopropenyl-α,α-dimethylbenzyl]carbodiimide is employed in an amount of 0.1 to 2 wt%, preferably 0.5 to 1 wt%, based on the total mixture.

11. Process according to Claims 9 and 10, **characterized in that** the bis[3-isopropenyl-α,α-dimethylbenzyl]carbodiimide is preferably meteredin in liquid form continuously or batchwise at temperatures of 20 - 50 °C, particularly preferably 25 - 35 °C.

12. Use of bis[3-isopropenyl-α,α-dimethylbenzyl]carbodiimide according to Claim 7 for hydrolysis inhibition.

## Revendications

1. Procédé de fabrication de bis[3-isopropényl-α,α-diméthylbenzyl]carbodiimide, **caractérisé en ce que** de l'isocyanate de 3-isopropényl-α,α-diméthylbenzyle est carbodiimidisé en présence de 0,1 à 20 % en poids de sels de césium basiques à des températures comprises entre 160 et 220 °C.

2. Procédé selon la revendication 1, **caractérisé en ce que** du carbonate de césium et/ou de l'alcoolate de césium, de préférence du méthylate de césium et/ou de l'éthylate de césium, sont utilisés en tant que sels de césium basiques.

3. Procédé selon la revendication 1 ou 2, **caractérisé en ce que** les sels de césium basiques sont éliminés par filtration après la carbodiimidisation.

4. Procédé selon l'une quelconque des revendications 1 à 3, **caractérisé en ce que** la carbodiimidisation a lieu dans un solvant.

5. Procédé selon la revendication 4, **caractérisé en ce que** des benzènes et/ou dibenzènes substitués une, deux, trois fois ou plus avec un alkyle, avec alkyle = C₁-C₃, sont utilisés en tant que solvant.

6. Procédé selon la revendication 5, **caractérisé en ce que** des xylènes sont utilisés en tant qu'alkylbenzènes.

7. Bis[3-isopropényl-α,α-diméthylbenzyl]carbodiimide pouvant être obtenu par un procédé selon l'une quelconque des revendications 1 à 6.

8. Stabilisateur contenant au moins 90 % en poids de bis[3-isopropényl-α,α-diméthylbenzyl]carbodiimide selon la revendication 7 et au plus 1 ppm de composés de phosphore hétérocycliques.

9. Procédé de fabrication de polyuréthanes, de préférence de polyuréthanes thermoplastiques, **caractérisé en ce que** la réaction des polyols avec les diisocyanates est réalisée en présence d'un bis[3-isopropényl-α,α-diméthylbenzyl]carbodiimide selon la revendication 7.

10. Procédé selon la revendication 9, **caractérisé en ce que** le bis[3-isopropényl-α,α-diméthylbenzyl]carbodiimide est utilisé en une quantité de 0,1 à 2 % en poids, de préférence de 0,5 à 1 % en poids, par rapport au mélange total.

11. Procédé selon les revendications 9 et 10, **caractérisé en ce que** le bis[3-isopropényl-α,α-diméthylbenzyl]carbodiimide est introduit de préférence sous forme liquide de manière continue ou discontinue à des températures de 20 à 50 °C, de manière particulièrement préférée de 25 à 35 °C.

12. Utilisation de bis[3-isopropényl-α,α-diméthylbenzyl]carbodiimide selon la revendication 7 pour la protection contre l'hydrolyse.
